**Europäisches Patentamt**

**European Patent Office**

**Office européen des brevets**

(19)

(11) Veröffentlichungsnummer: **0 067 355**

**B1**

(12)

# EUROPÄISCHE PATENTSCHRIFT

(45) Veröffentlichungstag der Patentschrift:
15.01.86

(21) Anmeldenummer: 82104774.3

(22) Anmeldetag: 01.06.82

(51) Int. Cl.⁴: **B 01 J 37/04,** C 07 C 120/14,
C 07 C 51/21

(54) Rieselfähige, katalytische Mischungen.

(30) Priorität: 13.06.81 DE 3123521

(43) Veröffentlichungstag der Anmeldung:
22.12.82 Patentblatt 82/51

(45) Bekanntmachung des Hinweises auf die Patenterteilung:
15.01.86 Patentblatt 86/3

(84) Benannte Vertragsstaaten:
AT DE GB IT NL

(56) Entgegenhaltungen:
DE-A-3 006 894
DE-B-1 000 120
FR-A-2 407 254
GB-A-978 520
GB-A-1 459 779
US-A-2 414 812
US-A-4 235 754

(73) Patentinhaber: EC ERDÖLCHEMIE GMBH, Postfach
75 20 02, D-5000 Köln 71 (DE)

(72) Erfinder: Ennenbach, Karl-Heinz, Ostpreussenallee
8, D-4047 Dormagen 1 (DE)
Erfinder: Haardt, Hans-Jürgen, Dr., Rurstrasse 3,
D-5024 Pulheim (DE)
Erfinder: Neeb, Ernst-Friedrich, Dr., Im
Kronenpuetzchen 28, D-4047 Dormagen 11 (DE)
Erfinder: Paris, Nikolaus, Ing., van-Liebig-Strasse
4, D-4047 Dormagen 1 (DE)

(74) Vertreter: Mann, Volker, Dr., c/o Bayer
Aktiengesellschaft Zentralbereich Patente
Marken und Lizenzen, D-5090 Leverkusen-
Bayerwerk (DE)

EP 0 067 355 B1

**0 067 355**

## Beschreibung

Die vorliegende Erfindung betrifft neue rieselfähige katalytische Mischungen für das Fließbett oder das wirbelbett, ein Verfahren zu ihrer Herstellung und ihre Verwendung.

Viele großtechnisch durchgeführte Oxidationsprozesse durch Umsetzung von Kohlenwasserstoffen mit Sauerstoff oder Sauerstoff enthaltenden Gasen, gegebenenfalls zusätzlich in Gegenwart von Ammoniak, werden in der Gasphase bei erhöter Temperatur durchgeführt, wobei das Gemisch der gasförmigen Einsatzstoffe an einem rieselfähigen Katalysator im Fließbett oder im Wirbelbett vorbeigeführt wird. Beispiele für solche Oxidationsverfahren sind die Umsetzung von Propylen zu Acrylsäure oder Acrolein, von Propylen in Gegenwart von Ammoniak zu Acrylnitril, von i-Buten zu Methacrylsäure oder Methacrolein, von i-Buten in Gegenwart von Ammoniak zu Methacrylnitril, von n-Butan zu Maleinsäureanhydrid, von Naphtalin oder o-Xylol zu Phthalsäureanhydrid oder die Oxidehydrierung von Isoamylenen oder Methylbutanolen zu Isopren.

Wegen der großen technischen Bedeutung dieser Verfahren ist eine große Anzahl katalytisch wirksamer Elemente, insbesondere in Form ihrer Oxide, ihrer Oxosalze, ihrer Mischoxide oder in Form von Mischungen ihrer Oxide untersucht worden. Hierbei wurde eine große Anzahl von Metallen, Halbmetallen oder Nichtmetallen aus fast allen Gruppen des Periodensystems der Elemente (Mendelejew) in den verschiedensten Kombinationen herangezogen. Beispielsweise seien die folgenden Elemente genannt: Eisen, Kobalt, Nickel, Mangan, Molybdän, Chrom, Wolfram, Uran, Wismut, Phosphor, Arsen, Antimon, Vanadin, Niob, Zinn, Zirkon, Blei, Bor, Aluminium, Tallium, Lantan, Cer, Magnesium, Calcium, Strontium, Barium, Zink, Cadmium, Kupfer, Silber, Lithium, Natrium, Kalium, Rubidium, Cäsium. Da diese Elemente oder Verbindungen und Mischungen hieraus im Zustand der Oxide oder der Oxosalze vorliegen, enthalten sie stets einen Sauerstoffgehalt, der sich an den Erfordernissen der Valenz orientiert. Rieselfähige Oxidationskatalysatoren, wie sie zum Stande der Technik gehören, können Aktivelemente aus der Zahl der o.g. in Form ihrer Oxide oder Oxosalze allein darstellen oder sie können auf einem inerten Material, wie Siliciumdioxid-Materialien oder Aiuminiumoxid-Materialien, aufgebracht sein.

Die bekannt gewordenen Oxidationskatalysatoren leiden unter dem Mangel, daß Umsatz und Selektivität der Ausgangsstoffe zu den Endprodukten unvollständig sind. Die mangelnde Selektivität bei gegebenem Umsatz ist hierbei im allgemeinen auf eine unerwünschte Weiteroxidation des organischen Einsatzmaterials zu Kohlendioxid und Wasser zu sehen.

Es wurden neue rieselfähige katalytische Mischungen für das Fließbett oder das Wirbelbett gefunden, die aus 50 bis 95 Gew.-% eines in feinkörniger Form vorliegenden Oxidationsträgerkatalysators und 50 bis 5 Gew.-% eines in Bezug auf die Oxidationskatalyse inerten feinkörningen Materials bestehen, dessen Kornspektrum im wesentlichen innerhalb der Grenzen des Kornspektrums des Oxidationskatalysators liegt oder sich unmittelbar an die Grenze des Kornspektrums des Oxidationskatalysators anschließt und dessen Dichte etwa 80 bis 120 % der Dichte des Oxidationskatalysators beträgt.

Als Träger für die Oxidationskatalysatoren in den erfindungsgemäßen Mischungen seien solche genannt, die üblicherweise als inerte Katalysatorträger Verwendung finden, wie $SiO_2$ und katalytisch inerte Silikate, $Al_2O_3$ in seinen verschiedenen katalytisch inerten Modifikationen, Carbonate, wie Calciumcarbonat, Bariumcarbonat oder Magnesiumcarbonat, Sulfate, wie Strontiumsulfat oder Bariumsulfat, oder katalytisch unwirksame Carbide oder Silicide, wie Carborundum. Das inerte Trägermaterial bildet im allgemeinen 20 bis 80 %, bevorzugt 30 bis 70 %, besonders bevorzugt 40 bis 60%,des Gesamtgewichts des Katalysators. Der jeweils verbleibende Rest zu 100 Gew.-% stellt das oder die katalytisch aktive(n) Elemente in ihrer oxidischen Form dar.

Als katalytisch aktive Elemente seien die oben erwähnten genannt, die in einer Vielzahl unterschiedlicher Zusammensetzungen in der technischen Literatur genannt sind und dem Fachmann bekannt sind. Als bevorzugte, katalytisch wirksame Elemente seien genannt: Eisen, Kobalt, Nickel, Mangan, Molybdän, Chrom, Wolfram, Tellur, Wismut, Phosphor, Antimon, Vanadin, Blei, Kupfer, Kalium und Cäsium. Beispielhaft seien Oxidationskatalysatoren genannt, die als hauptsächliche Aktivkomponente Molybdän, daneben als weitere Hauptkomponenten Wismut, Eisen, Kobalt und Nickel und als untergeordnete ergänzende Bestandteile Chrom, Mangan, sowie Alkalimetalle wie Kalium oder Cäsium, enthalten. In solchen Katalysatoren kann weiterhin eine geringe Menge Phosphor vorliegen. Als weiteres Beispiel seien Oxidationskatalysatoren mit den aktiven Hauptbestandteilen Antimon und Eisen, weiteren Hauptbestandteilen Tellur und Kupfer und untergeordneten Mengen Molybdän, Wolfram und Vanadin sowie gegebenenfalls Phosphor und Alkalimetallen genannt. Als noch weiteres Beispiel seien Oxidationskatalysatoren genannt, die als aktive Bestandteile lediglich Molybdän, Wismut, Phosphor und Eisen enthalten. Die letztgenannten können gegebenenfalls zusätzlich Blei enthalten. Alle Oxidationskatalysatoren enthalten weiterhin Sauerstoff in einer Menge, die der stöchiometrischen Notwendigkeit enspricht.

Die Herstellung von Oxidationsträgerkatalysatoren mit den genannten aktiven Bestandteilen ist dem Fachmann bekannt. Sie kann beispielsweise ausgeführt werden durch gemeinsames Ausfällen des Trägers und der aktiven Bestandteile aus einer Lösung oder Suspension dieser Bestandteile und anschließendes Calcinieren. Eine weitere Art der Herstellung besteht im Auftränken der aktiven Bestandteile auf bereits calcinierte und damit unlöslich gemachte Trägersubstanzen und anschließendes nochmaliges Calcinieren. Noch eine weitere, ebenfalls bekannte Herstellungsmethode besteht darin, Oxidationsträgerkatalysatoren, die bereits eine Reihe von aktiven Bestandteilen enthalten, zusätzlich mit Lösungen von weiteren aktiven Bestandteilen zu tränken oder zu besprühen und den derart behandelten Katalysator erneut zu calcinieren. Es ist dem Fachmann bekannt, durch Einstellen der Calciniertemperatur und der Calcinierzeit die innere Oberfläche des Oxidationsträgerkatalysators zu variieren und dadurch verschiedenartige katalytische Effekte hervorzurufen.

2

Die genannten Oxidationsträgerkatalysatoren für die erfindungsgemäßen Mischungen liegen in feinkörniger Form vor, beispielsweise mit Durchmessern von 10 bis 300 µm, bevorzugt 20 bis 180 µm. Die beschriebenen Oxidationsträgerkatalysatoren in feinkörniger Form bilden 50 bis 95 Gew.-%, bevorzugt 55 bis 85 Gew.-% der erfindungsgemäßen Mischungen. Der Rest zu 100 %, nämlich 50 bis 5 Gew.-%, bevorzugt 45 bis 15 Gew.-%, der erfindungsgemäßen Mischungen, ist ein in Bezug auf die Oxidationskatalyse inertes feinkörniges Material, dessen Kornspektrum im wesentlichen innerhalb der Grenzen des Kornspektrums des Oxidationskatalysators liegt oder sich unmittelbar an die Grenzen des Kornspektrums des Oxidationskatalysators anschließt. Als Kornspektrum seien beispielsweise, ebenso wie für die Oxidationskatalysatoren, 10 bis 300 µm, bevorzugt 20 bis 180 µm, genannt. Insbesondere für den Fall, daß das Kornspektrum des Oxidationskatalysators einen noch engeren Bereich, beispielsweise 40 bis 160 µm aufweist, kann sich das Kornspektrum des inerten feinkörnigen Materials an das genannte engere Kornspektrum des Oxidationskatalysators anschließen, beispielsweise an den unteren Bereich oder an den oberen Bereich. Hierfür seien beispielhaft Kornspektren von etwa 20 bis etwa 50µ oder etwa 160 bis etwa 250µ genannt. Die genannten Kornspektren des Oxidationskatalysators und des feinkörnigen inerten Materials können selbstverständlich auch an den beispielhaft genannten Grenzen von 40 bzw. 160µ überlappen. In bevorzugter Weise sei ein inertes feinkörniges Material genannt, das im wesentlichen im Bereich des Feinkornanteils oder im unmittelbaren Anschluß an den Feinkornbereich des Oxidationskatalysators liegt.

Erfindungsgemäß liegt die Dichte des inerten feinkörnigen Materials in der Nähe der Dichte des Oxidationskatalysators, beispielsweise bei 80 bis 120 %, bevorzugt 95 bis 105 %, besonders bevorzugt 97 bis 103 % der Dichte des Oxidationskatalysators.

Als katalytisch inertes Material für die erfindungsgemäßen Mischungen seien beispielsweise genannt: Alle o.g. Katalysatorträger, die für sich allein katalytisch nicht wirksam sind, sofern sie nach Korngröße oder Dichte erfindungsgemäß in Frage kommen. Besonders sei Seesand geeigneter Körnung und anderes $SiO_2$-Trägermaterial genannt. Überraschenderweise hat sich weiter gezeigt, daß sich desaktivierte und katalytisch nicht mehr oder nur in unzureichender Weise aktive Oxidationskatalysatoren als erfindungsgemäß zumischbares inertes feinkörniges Material eignen. Hierbei kann der zugemischte desaktivierte Oxidationskatalysator als Inertmaterial die gleiche oder eine andere chemische Zusammensetzung haben wie der in den erfindungsgemäßen Mischungen vorhandene aktive Oxidationskatalysator. In bevorzugter Weise wird jedoch ein desaktivierter, inerter Mischungsanteil gewählt, der die gleiche chemische Zusammensetzung wie der aktive Oxidationsträgerkatalysator hat. Die Desaktivierung wird durch Calcinieren des zugrundeliegenden Oxidationsträgerkatalysators bei einer Temperatur von 600 bis 1000° C, bevorzugt 750 bis 850° C während einer Zeit von 10 Minuten bis 5 Stunden, bevorzugt 30 Minuten bis 1 Stunde, durchgeführt. Das aus dieser Desaktivierung hervorgehende, nunmehr inerte Material als Zumischbestandteil der erfindungsgemäßen Mischungen, hat nach der Desaktivierung eine innere Oberfläche von beispielsweise 1 bis 15 m²/g, bevorzugt 2 bis 5 m²/g.

Die erfindungsgemäßen Mischungen zeigen überraschenderweise einen Anstieg der katalytischen Aktivität, obwohl durch die Zumischung von inerten Bestandteilen ein Verdünnungseffekt und damit ein Absinken der katalytischen Aktivität zu erwarten gewesen wäre. Die Erhöhung der katalytischen Aktivität zeigt sich in einer Erhöhung der Selektivität um etwa 2 bis zu 10 Absolut-Prozentpunkten, bevorzugt 2,5 bis zu 8 Absolut-Prozentpunkten, verglichen mit der vorher vorhandenen Selektivität des aktiven Oxidationsträgerkatalysators allein. Die Erhöhung der Selektivität bedeutet ein Zurückgehen der totalen Verbrennung des organischen Materials zu den unerwünschten Endprodukten $CO_2$ und $H_2O$. Die Erhöhung der Selektivität kann jedoch auch darin bestehen, daß mit einer verkleinerten absoluten Menge an Oxidationsträgerkatalysator unter erfindungsgemäßer Zumischung eines Inertbestandteiles kein Absinken, sondern ein Gleichbleiben der Aktivität beobachtet wird. Dies bedeutet also eine relative Steigerung der Aktivität. So wurde beispielsweise bei Zumischung von calciniertem $SiO_2$-Gel als Katalysatorträger in einer Menge von 20 % zu 80 % eines Oxidationsträgerkatalysators bei der Acrylnitril-Herstellung keine Ausbeutesteigerung, aber interessanterweise auch kein Ausbeuteabfall beobachtet. Dies bedeutet, bezogen auf den Oxidationsträgerkatalysator, eine Steigerung der Aktivität auf etwa 125 %, bezogen auf die Einsatzmenge des Katalysators.

Die beschriebenen Effekte der erfindungsgemäßen Mischungen werden besonders deutlich an Oxidationsträgerkatalysatoren zur Herstellung von Acrylnitril und/oder Methacrylnitril unter Zumischung einer Inertkomponente beobachtet. In besonders bevorzugter Weise ist hierbei die zugemischte Inertkomponente ein durch das beschriebene Calcinieren desaktivierter Oxidationsträgerkatalysator für die Herstellung von Acrylnitril und/oder Methacrylnitril. Es wird hierbei beobachtet, daß sowohl neue Ammonoxidationskatalysatoren eine zusätzliche Steigerung ihrer Selektivität erfahren als auch, daß gebrauchte Ammonoxidationskatalysatoren, deren Selektivität und Ausbeute abgesunken waren, auf die Aktivitäts- und Selektivitätswerte neuer Katalysatoren wieder angehoben werden können. Es ist unerheblich, ob der desaktivierte, inerte Zumischanteil aus einem frischen Ammonoxidationskatalysator oder einem bereits gebrauchten Ammonoxidationskatalysator durch die oben beschriebenen Maßnahmen hergestellt wird. Dies eröffnet die Möglichkeit zur Weiterverwendung bereits gebrauchter Oxidationskatalysatoren, besonders Ammonoxidationskatalysatoren, sowohl als aktiver Oxidationsträgerkatalysator als auch als desaktivierter inerter Zumischanteil im Rahmen der erfindungsgemäßen Mischungen.

Beispielsweise kann man hierzu so vorgehen, daß eine Teilmenge eines bereits gebrauchten Oxidationskatalysators durch erneutes Calcinieren nach den oben beschriebenen Bedingungen desaktiviert wird und mit einer weiteren Teilmenge nicht in dieser Weise behandelten Oxidationskatalysators zu erfindungsgemäßen Mischungen vereinigt wird. Man kann jedoch auch so vorgehen, daß von einem Oxidationskatalysator, besonders Ammonoxidationskatalysator, durch Sieben ein Teil des Kornspektrums abgetrennt wird. Dieser abgetrennte Teil des Kornspektrums wird dann in der oben beschriebenen Weise zur Desaktivierung calciniert und mit dem restlichen Kornspektrum des noch im aktiven Zustand verbliebenen Oxidationskatalysators zu einer erfindungsgemäßen Mischung vereint. Für den letzeren Fall ist das Aussieben des Feinkornanteils, Desaktivieren des Feinkornanteils in der beschriebenen Weise und Wiedervereinigung mit dem Grobkorn eine vielfach mögliche Variante für die Bereitung von erfindungsgemäßen Mischungen. Als auszusiebendes Feinkorn sei hierbei beispielsweise der Anteil unter 100 µm, bevorzugt unter 70 µm, besonders bevorzugt unter 50 µm, genannt.

Die Erfindung betrifft weiterhin ein Verfahren zur Herstellung einer rieselfähigen katalytischen Mischung aus 50 bis 95 Gew.-% eines in feinkörniger Form vorliegenden Oxidationsträgerkatalysators und 50 bis 5 Gew.-% eines in Bezug auf die Oxidationskatalyse inerten Materials, dessen Kornspektrum im wesentlichen innerhalb der Grenzen des Kornspektrums des Oxidationskatalysators liegt oder sich unmittelbar an die Grenzen des Kornspektrums des Oxidationskatalysators anschließt und dessen Dichte etwa 80 bis 120 % der Dichte des Oxidationskatalysators beträgt, das dadurch gekennzeichnet ist, daß man die genannten Mischungsbestandteile mechanisch mischt. Wie bereits beschrieben, wird bevorzugt als Zumischanteil ein durch die oben beschriebenen Maßnahmen desaktivierter Oxidationsträgerkatalysator eingesetzt. Wie ebenfalls bereits beschrieben, wird das erfindungsgemäße Verfahren zur Herstellung der erfindungsgemäßen rieselfähigen katalytischen Mischung bevorzugt durch Einsatz eines Ammonoxidationskatalysators und eines in der beschriebenen Weise desaktivierten Ammonoxidationskatalysators als der inerten Komponente durchgeführt.

Die Erfindung betrifft weiterhin die Verwendung der beschriebenen erfindungsgemäßen katalytischen Mischungen für das Fließbett oder das wirbelbett zur Herstellung von Acrylnitril und/oder Methacrylnitril nach dem Ammonoxidationsverfahren.

**Beispiele 1 bis 20**

(Beispiele 1, 3-5, 14 und 16 sind Vergleichsbeispiele)

In einem Laborreaktor von 1 m Höhe und 38,4 mm Durchmesser werden 550 g der in Tabelle I angegebenen Katalysatoren vorgelegt (Ausnahme Beispiel 4) und als Fließbett von unten nach oben unter den in Tabelle II angegebenen Bedingungen zur Acrylnitril-Herstellung eingesetzt. Als Kontaktbelastung ist kg Einsatzgase pro kg Katalysator pro Stunde zu verstehen. Die Verweilzeit beträgt etwa 7,5 bis 8,5 Sekunden, der Kopfdruck des Reaktors 1,8 bar. Zur Einstellung eines stationären Zustandes laufen alle Versuche mindestens 24 Stunden (vgl. Tabelle II); alle Versuche, auch die länger laufenden, werden ohne Aktivitätseinbuße abgebrochen. Die Ergebnisse zeigt Tabelle I.

Als Katalysatoren kommen zum Einsatz:

Katalysator A der Zusammensetzung (als Atomzahlen):

$Mo_{12}Co_4Fe_2Ni_2Bi_1(Cr,Mn,K,Cs)_1O_x$, 50-gew.-%ig auf $SiO_2$ als Träger (x = der Stöchiometrie entsprechend); Kornverteilung: 43 Gew.-% < 45 µm; 57 Gew.-% 45-160 µm;

Katalysator B wie A, jedoch nach 1-jährigem Gebrauch zur Acrylnitril-Herstellung; Kornverteilung: 26 Gew.-% < 45 µm; 74 Gew.-% 45-160µm; Katalysator C der Zusammensetzung (als Atomzahlen):

$Mo_{11}Co_{3,8}Fe_{2,7}Ni_2Bi_1(P,K)_{0,5}O_x$, 50-gew.-%ig auf $SiO_2$; nach 4-jährigem Gebrauch zur Acrylnitril-Herstellung; Kornverteilung 13,5 Gew.-% < 45 µm; 86,5 Gew.-% 45-160 µm

Katalysator D der Zusammensetzung (als Atomzahlen):

$Sb_{20}Fe_{12}Te_4Cu_{3,5}(Mo,W,V)_1O_x$, 50-gew.-%ig auf $SiO_2$; vorher nicht benutzter Katalysator; Kornverteilung: 35 Gew.-% < 45 µm; 65 Gew.-% 45-160 µm

Tabelle I

Alle Werte in Kohlenstoffprozent, hochgerechnet auf 100 % Kohlenstoffbilanz

| Katalysatoren | ACN | HCN | $CO_2 + CO$ | Selektivität |
|---|---|---|---|---|
| 1) Katalysator C | 71,5 % | 10,2 % | 15,4 % | 72,2 % |
| 2) Das Feinkorn (25 %) < 45 um des Katalysators C wurde ausgesiebt, 1 h bei 800°C calciniert und anschl. mit 75 % Grobkorn gemischt | 77,4 % | 8,1 % | 11,1 % | 78,5 % |
| 3) Katalysator B | 74,0 % | 7,4 % | 14,5 % | 74,6 % |
| 4) Katalysator B: Der Test wurde mit 82 % der Katalysator-Sollfüllung durchgeführt | 73,3 % | 7,1 % | 14,9 % | 74,2 % |
| 5) Katalysator B: Der Katalysator wurde ausgesiebt und wie folgt ein Kornspektrum zusammengestellt, welches dem Ø-Kornspektrum des (frischen) Katalysators A entspricht (41,1 % < 45 um; 8,7 % 45-56 um; 17,5 % 56-71   19,8 % 71-90   3,8 % 90-100 um; 8,8 % 100-160 um) | 72,4 % | 8,7 % | 16,7 % | 72,5 % |

Tabelle I (Fortsetzung)

| Katalysatoren | ACN | HCN | $CO_2$ + CO | Selektivität |
|---|---|---|---|---|
| 6) Katalysator B:<br>Das Feinkorn (26,3 %) < 45 µm wurde aus-<br>gesiebt, 1 h bei 800°C calciniert und<br>anschließend mit dem Grobkorn gemischt | 78,8 % | 6,55 % | 10,7 % | 79,8 % |
| 7) Katalysator B:<br>Das Feinkorn < 45 µm wurde ausgesiebt,<br>1 h bei 800°C calciniert und anschl.<br>mit 60 % Grobkorn gemischt | 78,9 % | 6,6 % | 10,7 % | 79,8 % |
| 8) Katalysator B:<br>75 % Grobkorn > 45 µm wurden mit 25 %<br>30 min bei 850°C calciniertem Grob-<br>korn gemischt | 78,6 % | 5,2 % | 11,9 % | 80,0 % |

In den Beispielen 9-13 wurden folgende Katalysatormischen hergestellt. Jeweils 75 % gebrauchter Katalysator B wurden mit 25 % bei
unterschiedlichen Temperaturen und Verweilzeiten calciniertem Katalysator B gemischt.

| Katalysatoren | ACN | HCN | $CO_2$ + CO | Selektivität |
|---|---|---|---|---|
| 9) Calcinierter Anteil: 1 h bei 700°C cal. | 80,3 % | 4,5 % | 11,8 % | 81,1 % |
| 10) Calcinierter Anteil: 1 h bei 800°C cal. | 80,9 % | 5,0 % | 10,2 % . | 82,1 % |
| 11) Calcinierter Anteil: 1 h bei 850°C cal. | 80,2 % | 5,5 % | 9,85% | 81,3 % |
| 12) Calcinierter Anteil: 0,5 h bei 850°C cal. | 79,9 % | 6,0 % | 10,1 % | 81,0 % |
| 13) Calcinierter Anteil: 1 h bei 900°C cal. | 80,6 % | 5,5 % | 9,65% | 81,75% |

0 067 355

Tabelle I   (Fortsetzung)

| Katalysatoren | ACN | HCN | $CO_2$ + CO | Selektivität |
|---|---|---|---|---|
| 14) Katalysator A: | 79,0 % | 2,8 % | 15,06 % | 79,3 % |
| 15) Das Feinkorn 43,2 % <45 µm des Katalysators A wurde ausgesiebt, 1 h bei 800°C calciniert und anschließend mit dem unbehandelten Grobkorn gemischt | 82,0 % | 2,3 % | 10,5 % | 84,2 % |
| 16) Katalysator D: | 77,6 % | 5,8 % | 14,6 % | 78,8 % |
| ·17) 75 % Katalysator D wurde mit 25 % 30 min bei 850°C calciniertem frischem Katalysator D gemischt | 78,6 % | 4,5 % | 13,4 % | 81,1 % |

In den Beispielen 18-20 wurde Katalysator B (1 Jahr Laufzeit) in verschiedenen Konzentrationen mit calciniertem Feinkorn < 45 µm (1 h bei 800°C) des Katalysators C gemischt.

| | ACN | HCN | $CO_2$ + CO | Selektivität |
|---|---|---|---|---|
| 18) Mischung mit 15 % Katalysator C < 45 µm 1 h bei 800°C calciniert | 77,2 % | 6,8 % | 12,7 % | 77,7 % |
| 19) Mischung mit 20 % Katalysator C < 45 µm 1 h bei 800°C calciniert | 77,4 % | 7,3 % | 12,1 % | 77,9 % |
| · 20) Mischung mit 25 % Katalysator C < 45 µm 1 h bei 800°C calciniert | 78,0 % | 7,3 % | 11,75% | 78,8 % |

## Tabelle II

| Beispiele | Kontaktbe-lastung | Molverhältnis Luft | : | $NH_3$ | : | Propylen | Temperatur | Versuchsdauer | |
|---|---|---|---|---|---|---|---|---|---|
| 1 | 0,045 | 10,4-10 | : | 1,23-1,2 | : | 1 | 425°C | 2 | Tage |
| 2 | 0,045 | 10,15-9,64 | : | 1,25 | : | 1 | 430°C | 3 | Tage |
| 3 | 0,045-0,05 | 10,3-10 | : | 1,23-1,15 | : | 1 | 425-435°C | 3 | Tage |
| 4 | 0,055 | 10,0-9,8 | : | 1,15-1,12 | : | 1 | 425-430°C | 2 | Tage |
| 5 | 0,045-0,048 | 10,9-10,4 | : | 1,2 | : | 1 | 425-430°C | 3 | Tage |
| 6 | 0,045 | 10-9,5 | : | 1,2-1,12 | : | 1 | 425-430°C | 5 | Tage |
| 7 | 0,045 | 9,9-9,65 | : | 1,25-1,15 | : | 1 | 425-435°C | 11 | Tage |
| 8 | 0,05 | 10,8-9,25 | : | 1,3-1,22 | : | 1 | 445°C | 2 | Tage |
| 9 | 0,05 | 11,1-9,7 | : | 1,45-1,27 | : | 1 | 445°C | 2 | Tage |
| 10 | 0,05 | 10-9,5 | : | 1,33-1,2 | : | 1 | 445°C | 2 | Tage |
| 11 | 0,05 | 9,6-9,5 | : | 1,27-1,25 | : | 1 | 445°C | 2 | Tage |
| 12 | 0,05 | 9,8-9,65 | : | 1,3-1,25 | : | 1 | 445°C | 13 | Tage |
| 13 | 0,05 | 9,7-9,55 | : | 1,27 | : | 1 | 445°C | 2 | Tage |
| 14 | 0,045 | 11,7 | : | 1,4 | : | 1 | 430°C | 1 | Tag |
| 15 | 0,045 | 11,25-10,95 | : | 1,4-1,65 | : | 1 | 430°C | 2 | Tage |
| 16 | 0,045 | 12,2-12,0 | : | 1,1-1,07 | : | 1 | 430-440°C | 5 | Tage |

Tabelle II

| Beispiele | Kontaktbe-lastung | Molverhältnis Luft | : | NH$_3$ | : | Propylen | Temperatur | Versuchsdauer |
|---|---|---|---|---|---|---|---|---|
| 17 | 0,045 | 12,1-11,7 | : | 1,1-1,07 | : | 1 | 435°C | 3 Tage |
| 18 | 0,045 | 10,0-9,7 | : | 1,17-1,15 | : | 1 | 430-435°C | 7 Tage |
| 19 | 0,045 | 9,85-9,7 | : | 1,25-1,2 | : | 1 | 430°C | 6 Tage |
| 20 | 0,045 | 10,0-9,7 | : | 1,27 | : | 1 | 430°C | 3 Tage |

0 067 355

**Patentansprüche**

1) Rieselfähige katalytische Mischungen, für das Flisßbett oder das wirbelbett, bestehend aus 50 bis 95 Gew.-% eines in feinkörniger Form vorliegenden Oxidationsträgerkatalysators und 50 bis 5 Gew.-% eines in Bezug auf die Oxidationskatalyse inerten feinkörnigen Materials, dessen Kornspektrum im wesentlichen innerhalb der Grenzen des Kornspektrums des Oxidationskatalysators liegt oder sich unmittelbar an die Grenzen des Kornspektrums des Oxidationskatalysators anschließt und dessen Dichte etwa 80 bis 120 % der Dichte des Katalysators beträgt.

2) Katalytische Mischung nach Anspruch 1, bestehend aus 55 bis 85 Gew.-% des Oxidationskatalysators und 45 bis 15 Gew.-% des inerten Materials.

3) Katalytische Mischung nach Ansprüchen 1 und 2, dadurch gekennzeichnet, daß der Oxidationskatalysator ein bereits gebrauchter Oxidationskatalysator ist.

4) Katalytische Mischung nach Ansprüchen 1 bis 3, dadurch gekennzeichnet, daß der Oxidationskatalysator ein Ammonoxidationskatalysator ist.

5) Katalytische Mischung nach Ansprüchen 1 bis 4, dadurch gekennzeichnet, daß das inerte Material ein durch Calcinieren desaktivierter Oxidationskatalysator mit einer inneren Oberfläche von höchstens 15 m²/g ist.

6) Katalytische Mischung nach Anspruch 5, dadurch gekennzeichnet, daß ein desaktiviertes Material eingesetzt wird, das durch Calcinieren eines Oxidationskatalysators bei 600 bis IOOO°C für 10 Minuten bis 5 Stunden erhalten wird.

7) Katalytische Mischung nach Ansprüchen 1 bis 6, dadurch gekennzeichnet, daß das Kornspektrum des inerten Materials im wesentlichen im Bereich des Feinkornanteils oder im unmittelbaren Anschluß an den Feinkornbereich des Oxidationskatalysators liegt.

8) Katalytische Mischung nach Ansprüchen 1 bis 7, dadurch gekennzeichnet, daß die Dichte des inerten Materials bei 95 bis 105 % der Dichte des Oxidationskatalysators liegt.

9) Verfahren Zur Herstellung einer rieselfähigen katalytischen Mischung für das Fließbett oder das wirbelbett, bestehand aus 50 bis 95 Gew.-% eines in feinkörniger Form vorliegenden Oxidationsträgerkatalysators und 50 bis 5 Gew.-% eines in Bezug auf die Oxidationskatalyse inerten Materials, dessen Kornspektrum im wesentlichen innerhalb der Grenzen des Kornspektrums des Oxidationskatalysators liegt oder sich unmittelbar an die Grenzen des Kornspektrums des Oxidationskatalysators anschließt und dessen Dichte bei etwa 80 bis 120 % der Dichte des Oxidationskatalysators beträgt, dadurch gekennzeichnet, daß man die genannten Mischungsbestandteile mechanisch mischt.

10) Verwendung einer katalytischen Mischung für das Fließbett oder das wirbelbett nach Anspruch 1 zur Herstellung von (Meth)Acrylnitril nach dem Ammonoxidationsverfahren.

**Claims**

1) Free-flowing catalytic mixtures for moving beds or fluidised beds, consisting of 50 to 95% by weight of a finely divided supported oxidation catalyst and 50 to 5% by weight of a finely divided material which is inert in respect of oxidation catalysis, the particle-size spectrum of which is principally within the limits of the particle-size spectrum of the oxidation catalyst or is directly contiguous to the limits of the particle-size spectrum of the oxidation catalyst, and the density of which is about 80 to 120% of the density of the catalyst.

2) Catalytic mixture according to Claim 1, consisting of 55 to 85% by weight of the oxidation catalyst and 45 to 15% by weight of the inert material.

3) Catalytic mixture according to Claims 1 and 2, characterised in that the oxidation catalyst is an oxidation catalyst which has already been used.

4) Catalytic mixture according to Claims 1 to 3, characterised in that the oxidation catalyst is an ammonoxidation catalyst.

5) Catalytic mixture according to Claims 1 to 4, characterised in that the inert material is an oxidation catalyst, deactivated by calcination, with an internal surface area of at most 15 m²/g.

6) Catalytic mixture according to Claim 5, characterised in that a deactivated material is used which is obtained by calcination of an oxidation catalyst at 600 to 1,000°C for 10 minutes to 5 hours.

7) Catalytic mixture according to Claims 1 to 6, characterised in that the particle-size spectrum of the inert material lies principally in the range of the fine particle fraction or in direct contact with the fine particle range of the oxidation catalyst.

8) Catalytic mixture according to Claims 1 to 7, characterised in that the density of the inert material is 95 to 105% of the density of the oxidation catalyst.

9) Process for the preparation of a free-flowing catalytic mixture for moving beds or fluidised beds, consisting of 50 to 95% by weight of a finely divided supported oxidation catalyst and 50 to 5% by weight of a material inert in respect of oxidation catalysis, the particle-size spectrum of which lies principally within the limits of the particle-size spectrum of the oxidation catalyst or is directly contiguous to the limits of the particle-size spectrum of the oxidation catalyst, and the density of which is about 80 to 120% of the density of the oxidation catalyst, characterised in that the said constituents of the mixture are mechanically mixed.

10) Use of a catalytic mixture for moving beds or fluidised beds according to Claim 1 for the preparation of (meth)acrylonitrile by the ammonoxidation process.

## Revendications

1. Mélanges catalytiques aptes à l'écoulement pour lit fluide ou pour lit tourbillonnaire, composés de 50 à 95% en poids d'un catalyseur d'oxydation fixé sur un support, se présentant sous la forme de grains de petit diamètre, et de 50 à 5% en poids d'une matière en grains de petit diamètre, inerte vis-à-vis de la catalyse d'oxydation, dont la granulométrie se situe principalement entre les limites de la granulométrie du catalyseur d'oxydation ou se rattache directement aux limites de la granulométrie du catalyseur d'oxydation et dont la densité s'élève à environ 80-120% de la densité du catalyseur.

2. Mélange catalytique suivant la revendication 1, composé de 55 à 85% en poids du catalyseur d'oxydation et de 45 à 15% en poids de la matière inerte.

3. Mélange catalytique suivant les revendications 1 et 2, caractérisé en ce que le catalyseur d'oxydation est un catalyseur d'oxydation qui a déjà servi.

4. Mélange catalytique suivant les revendications 1 à 3, caractérisé en ce que le catalyseur d'oxydation est un catalyseur d'ammonoxydation.

5. Mélange catalytique suivant les revendications 1 à 4, caractérisé en ce que la matière inerte est un catalyseur d'oxydation désactivé par calcination ayant une surface interne au maximum égale à 15 m$^2$/g.

6. Mélange catalytique suivant la revendication 5, caractérisé en ce qu'on utilise une matière désactivée qui est obtenue par calcination d'un catalyseur d'oxydation à 600-1000°C pendant 10 minutes à 5 heures.

7. Mélange catalytique suivant les revendications 1 à 6, caractérisé en ce que la granulométie de la matière inerte se situe principalement dans le domaine de la fraction à grains fins ou se raccorde directement au domaine de la fraction à grains fins du catalyseur d'oxydation.

8. Mélange catalytique suivant les revendications 1 à 7, caractérisé en ce que la densité de la matière inerte se situe à 85-105% de la densité du catalyseur d'oxydation.

9. Procédé de production d'un mélange catalytique apte à s'écouler pour lit fluide ou pour lit tourbillonnaire, composé de 50 à 95% en poids d'un catalyseur d'oxydation fixé sur un support, se présentant sous une forme à grains fins, et de 50 à 5% en poids d'une matière inerte vis-à-vis de la catalyse d'oxydation, dont la granulométrie se situe principalement entre les limites de celle du catalyseur d'oxydation ou se rattache directement aux limites de la granulométrie du catalyseur d'oxydation et dont la densité s'élève à environ 80-120% de celle du catalyseur d'oxydation, caractérisé en ce qu'on mélange mécaniquement les composants mentionnés du mélange.

10. Utilisation d'un mélange catalytique pour lit fluide ou pour lit tourbillonnaire selon la revendication 1 pour la production de (méth)acrylonitrile par le procédé d'ammonoxydation.